# EUROPEAN PATENT SPECIFICATION

(11) **EP 2 687 222 B1**
(45) Date of publication and mention of the grant of the patent: **27.02.2019**
(21) Application number: 12757563.7
(22) Date of filing: 14.03.2012
(51) Int. Cl.: A61K 36/73, A61K 8/34, A61K 8/46, A61K 8/97, A61K 31/047, A61K 31/197, A61K 36/23, A61K 36/53, A61K 36/75, A61P 17/16, A61P 37/08, A61P 43/00, A61Q 19/00

(54) **BLEOMYCIN HYDROLASE PRODUCTION PROMOTOR**
PROMOTOR EINER BLEOMYCIN-HYDROLASE-HERSTELLUNG
PROMOTEUR DE PRODUCTION DE BLÉOMYCINE HYDROLASE

(30) Priority: 15.03.2011 JP 2011057126
(43) Date of publication of application: 22.01.2014
(60) Divisional application: 19151688.9
(73) Proprietor: Shiseido Company, Ltd., Chuo-ku Tokyo 104-8010 (JP)
(72) Inventor: HIBINO, Toshihiko, Yokohama-shi, Kanagawa 236-8643 (JP); YAMADA, Shoko, Yokohama-shi, Kanagawa 236-8643 (JP); FUKUSHIMA, Hidekazu, Yokohama-shi, Kanagawa 224-8558 (JP)
(74) Representative: Hart-Davis, Jason
(86) International application number: PCT/JP2012/056581
(87) International publication number: WO 2012/124738

(56) References cited:
- WO-A1-2008/114732
- WO-A1-2009/104118
- WO-A1-2009/142268
- JP-A- 49 085 244
- JP-A- 2000 212 027
- JP-A- 2005 206 539
- JP-A- 2005 239 609
- JP-A- 2006 016 337
- JP-A- 2009 143 898
- JP-A- 2010 105 980
- Y. KAMATA ET AL: "Bleomycin Hydrolase Is Regulated Biphasically in a Differentiation- and Cytokine-dependent Manner: RELEVANCE TO ATOPIC DERMATITIS", JOURNAL OF BIOLOGICAL CHEMISTRY, vol. 286, no. 10, 11 March 2011 (2011-03-11), pages 8204-8212, XP055134613, ISSN: 0021-9258, DOI: 10.1074/jbc.M110.169292
- MAMEDOV N ET AL: "Medicinal plants used in Russia and Central Asia for the treatment of selected skin conditions", JOURNAL OF HERBS, SPICES AND MEDICINAL PLANTS, FOOD PRODUCTS PRESS, BINGHAM, NY, US, vol. 11, no. 1-2, 1 January 2004 (2004-01-01), pages 191-222, XP009087352, ISSN: 1049-6475, DOI: 10.1300/J044V11N01_07
- DATABASE WPI Week 200235 Thomson Scientific, London, GB; AN 2002-310733 XP002728510, & JP 2001 354579 A (LION CORP) 25 December 2001 (2001-12-25)
- DATABASE WPI Week 200632 Thomson Scientific, London, GB; AN 2006-301858 XP002728511, & JP 2006 111560 A (NIPPON MENARD KESHOHIN KK) 27 April 2006 (2006-04-27)
- DATABASE BIOSIS [Online] BIOSCIENCES INFORMATION SERVICE, PHILADELPHIA, PA, US; 2004, KOBAYASHI H ET AL: "The effects of Hochu-ekki-to in patients with atopic dermatitis resistant to conventional treatment", XP002728512, Database accession no. PREV200500169020 & INTERNATIONAL JOURNAL OF TISSUE REACTIONS, vol. 26, no. 3-4, 2004, pages 113-117, ISSN: 0250-0868
- K.L.E. HON ET AL: "Efficacy and tolerability of a Chinese herbal medicine concoction for treatment of atopic dermatitis: a randomized, double-blind, placebo-controlled study", BRITISH JOURNAL OF DERMATOLOGY, vol. 157, no. 2, 1 August 2007 (2007-08-01), pages 357-363, XP055134678, ISSN: 0007-0963, DOI: 10.1111/j.1365-2133.2007.07941.x
- DATABASE MEDLINE [Online] US NATIONAL LIBRARY OF MEDICINE (NLM), BETHESDA, MD, US; April 2011 (2011-04), TAKANO NORIKAZU ET AL: "[Effects of ethanol extracts of herbal medicines on dermatitis in an atopic dermatitis mouse model].", XP002728513, Database accession no. NLM21467798 & TAKANO NORIKAZU ET AL: "[Effects of ethanol extracts of herbal medicines on dermatitis in an atopic dermatitis mouse model].", YAKUGAKU ZASSHI : JOURNAL OF THE PHARMACEUTICAL SOCIETY OF JAPAN APR 2011, vol. 131, no. 4, April 2011 (2011-04), pages 581-586, ISSN: 1347-5231

### SEQUENCE LISTING

<110> SHISEIDO COMPANY, LTD.
<120> Agent for Promoting Production of Bleomycin Hydrolase
<130> Z787-PCT
<150> JP2011-057126
   <151> 2011.03.15
<160> 16
<170> PatentIn version 3.5
<210> 1
   <211> 20
   <212> DNA
   <213> Artificial Sequence
<220>
   <223> G3PDH Forward primer
<400> 1
   tgtggtttgg ctgtgatgtt 20
<210> 2
   <211> 20
   <212> DNA
   <213> Artificial Sequence
<220>
   <223> G3PDH Reverse primer
<400> 2
   gcaccatcct gatcatcctt 20
<210> 3
   <211> 29
   <212> DNA
   <213> Artificial Sequence
<220>
   <223> BH gene specific primer 1
<400> 3
   tccctcgagt ctgtatcaga gcagctaca 29
<210> 4
   <211> 28
   <212> DNA
   <213> Artificial Sequence
<220>
   <223> BH gene specific primer 2
<400> 4
   tgaacacgcg tccgagctgc tcatggcg 28
<210> 5
   <211> 26
   <212> DNA
   <213> Artificial Sequence
<220>
   <223> BH specific primer containing Kpn 1
<400> 5
   ccgggtacca tcagagttcc ttagaa 26
<210> 6
   <211> 28
   <212> DNA
   <213> Artificial Sequence
<220>
   <223> BH specific primer containing Mlu 1
<400> 6
   taaatacgcg ttggcgccca cgctgccg 28
<210> 7
   <211> 24
   <212> DNA
   <213> Artificial Sequence
<220>
   <223> Sp-1 site forward primer
<400> 7
   ggaccccgtt tcagcctccc cgcc 24
<210> 8
   <211> 24
   <212> DNA
   <213> Artificial Sequence
<220>
   <223> Sp-1 site reverse primer
<400> 8
   ggcggggagg ctgaaacggg gtcc 24
<210> 9
   <211> 29
   <212> DNA
   <213> Artificial Sequence
<220>
   <223> MZF-1 site forward primer
<400> 9
   gactcagcaa cgcggttttg tccctccgc 29
<210> 10
   <211> 30
   <212> DNA
   <213> Artificial Sequence
<220>
   <223> MZF-1 site reverse primer
<400> 10
   gcggagggac aaaaccgcgt tgctgagtca 30
<210> 11
   <211> 22
   <212> DNA
   <213> Artificial Sequence
<220>
   <223> IRF-1/2 site forward primer
<400> 11
   gccgccgagc ctccggcgct cc 22
<210> 12
   <211> 22
   <212> DNA
   <213> Artificial Sequence
<220>
   <223> IRF-1/2 site reverse primer
<400> 12
   ggagcgccgg aggctcggcg gc 22
<210> 13
   <211> 20
   <212> DNA
   <213> Artificial Sequence
<220>
   <223> Forward Primer for BH
<400> 13
   tgtggtttgg ctgtgatgtt 20
<210> 14
   <211> 20
   <212> DNA
   <213> Artificial Sequence
<220>
   <223> Reverse Primer for BH
<400> 14
   gcaccatcct gatcatcctt 20
<210> 15
   <211> 30
   <212> DNA
   <213> Artificial Sequence
<220>
   <223> Forward Primer for GAPDH
<400> 15
   ggtgaaggtc ggagtcaacg gatttggtcg 30
<210> 16
   <211> 30
   <212> DNA
   <213> Artificial Sequence
<220>
   <223> Reverse Primer for GAPDH
<400> 16
   tattggaaca tgtaaaccat gtagttgagg 30

## Description

### TECHNICAL FIELD

The present invention provides a bleomycin hydrolase production promoter substrate for use in a method for improving or preventing dry skin caused by atopic dermatitis due to the reduction of bleomycin expression in skin.

### BACKGROUND ART

Keratin fibers of the epidermal granular layer aggregate by binding to a protein referred to as filaggrin during keratinization, creating a specific form referred to as a "keratin pattern". Although a precursor of filaggrin, profilaggrin (comprising 10 to 12 tandemly repeated filaggrin units) is present in large amounts in the keratohyalin granules of granule cells, during keratinization, keratin fibers are aggregated by dephosphorylation together with the formation of filaggrin monomers. Subsequently, after the aggregated fibers have been deiminated by the action of an enzyme known as peptidyl arginine deiminase (PAD) and released from keratin, they are degraded into amino acids and the like in the upper horny layer. These amino acids are referred to as natural moisturizing factors (NMF), and are known to play an important role in retaining moisture in the horny layer and absorbing ultraviolet rays (Blank, I.H., J. I. Dermatol., 18, 433 (1952); Blank, I.H., J. I. Dermatol., 21, 259 (1953)).

Ever since amino acids serving as the main component of NMF were clearly determined to originate in filaggrin, research has proceeded on the correlation between pathological states presenting with dry skin and filaggrin. In recent years, amino acids have been
clearly determined to decrease in dry skin associated with senile xerosis, atopic diseases and the like (Horii, I., et al., Br. J. Dermatol., 121, 587-592 (1989); Tanaka, M., et al., Br. J. Dermatol., 139, 618-621 (1989)).

PAD induces deimination of filaggrin by acting on the arginine residue thereof and converting it to a citrulline residue. It is considered that as a result of filaggrin being deiminated in this manner, affinity between filaggrin and keratin fibers weakens and the keratin fibers are released, thereby resulting in filaggrin being more susceptible to the action of proteases, which is ultimately degraded into NMF.

The inventors of the present invention identified calpain-1 as an enzyme that degrades filaggrin following its deimination by PAD, and determined that the degradation products thereof in the form of small peptide fragments are degraded into amino acid units, namely NMF, by bleomycin hydrolase (BH) (Journal of Investigative Dermatology (2008), Volume 128, Abstracts, S90, 539; Joint Conference of the 30th Annual Meeting of the Molecular Biology Society of Japan and the 80th Conference of the Japanese Biochemical Society, Collection of Presentation Abstracts, p. 583; Journal of Biological Chemistry, 284, No. 19, pp. 12829-12836, 2009, 30P-0251; and, Japanese Unexamined Patent Publication No. 2008-135944 (to be referred to as Application No. 944).

According to more recent research, some atopic dermatitis is known to occur due to an abnormality of the profilaggrin gene, and abnormalities of this gene are observed in roughly 5% to 50% of atopic dermatitis patients (Smith, F.J.D., et al., Nat. Genet. 38:337-42 (2006); Aileen Sandilands, et al., J. I. Dermatol., 127, 1282-1284 (2007); and, Nomura, T., et al., J. I. Dermatol., 128(6):1436-41 (2008)). However, this does not necessarily mean that the expression of filaggrin decreases dramatically in the skin of atopic dermatitis patients.

Japanese Patent Application JP2006-111560 A discloses a substance comprising one or more kinds selected from extracts of Oryza sativa L., Pueraria lobata ohwii, Prunus armeniaca L. var. ansu Maximowicz, Lonicera japonica Thunberg, Saxifraga stolonifera, Rubus suavissimus Shugan Lee, Apocynum venetum L., Crataegus cuneata, Rosa roxburghii Traff. f. normalis Rehd. et Wils, Acanthopanax senticosus Harms, Zizyphus jujuba var. inermis Rehd, Perilla frutescens crispa, Coptis japonica, Asarum sieboldii, Scutellaria baicalensis Georgi, Phellodendron amurense, Morus alba, Paeonia suffruticosa Andrews Paeonia, Paeonia lactiflora Pallas, Citrus unshiu Mercov, Sapindus mukorossi Gaertn, Syzygium aromaticum, Lilium candidum, Glycine max Merrill and Tremella fuciformis B.

### Prior Art Documents

### Non-Patent Documents

Non-Patent Document 1: Blank, I.H., J. I. Dermatol., 18, 433 (1952)
Non-Patent Document 2: Blank, I.H., J. I. Dermatol., 21, 259 (1953)
Non-Patent Document 3: Horii, I., et al., Br. J. Dermatol., 121, 587-592 (1989)
Non-Patent Document 4: Tanaka, M., et al., Br. J. Dermatol., 139, 618-621 (1989)
Non-Patent Document 5: Kamata, et al., J. Biochem., 141, 69-76, 2007
Non-Patent Document 6: Journal of Investigative Dermatology (2008), Volume 128, Abstracts, S90, 539
Non-Patent Document 7: Joint Conference of the 30th Annual Meeting of the Molecular Biology Society of Japan and the 80th Conference of the Japanese Biochemical Society, Collection of Presentation Abstracts, p. 583 3P-0251
Non-Patent Document 8: Journal of Biological Chemistry, 284, No. 19, pp. 12829-12836, 2009
Non-Patent Document 9: Smith, F.J.D., et al., Nat. Genet. 38:337-42 (2006)
Non-Patent Document 10: Aileen Sandilands, et al., J. I. Dermatol., 127, 1282-1284

### Patent Document

JP2006-111560 A

### DISCLOSURE OF THE INVENTION

### Problems to be Solved by the Invention

An object of the present invention is to provide a bleomycin hydrolase production promotor.

In the aforementioned Application No. 944, the inventor of the present invention clearly determined that promotion of the activity of bleomycin hydrolase improves the barrier function of skin via the production of NMF. In this manner, bleomycin hydrolase is thought to act in the final stage of NMF production. However, it is interesting to note that, since expression of filaggrin continues to be observed in numerous atopic dermatitis patients with respect to dry skin caused by atopic dermatitis, this action is thought to be caused by a different factor than an abnormality of the filaggrin gene.

The inventors of the present invention examined fluctuations in the expression of bleomycin hydrolase according to a dry skin test in human subjects and analyzed the mechanism for controlling that expression based on the hypothesis that decreased expression of bleomycin hydrolase in human skin is not only related to a decrease in the skin's barrier function caused by an abnormality of the NMF production mechanism, but also is related to atopic dermatitis mainly caused by immune disorders and dry skin and the like caused by that dermatitis. As a result, the inventors of the present invention found that decreased expression of bleomycin hydrolase is related to dry skin caused by atopic dermatitis, and that a control region that prominently induces expression of that enzyme is present in the 5'-flanking region of the gene that encodes that enzyme. More specifically, the inventors of the present invention cloned the 5'-flanking region of bleomycin hydrolase (BH). In a deletion analysis thereof, a region important for BH promoter activity was identified -216 bp upstream therefrom. An electrophoretic mobility shift assay demonstrated that MZF-1, Sp-1 and interferon regulator factors (IRF)-1/2 are able to bind to this region in vitro. Moreover, BH promoter activity decreased considerably by site-specific mutagenesis of the MZF-1 and Sp-1 motifs. These data suggested that BH expression is up-regulated via MZF-1 and Sp-1. It is interesting to note that the Th1 cytokine, interferon (IFN)-γ significantly decreases expression of BH. Inhibitory effects of IFN-γ on BH expression were verified in an analysis using site-specific mutagenesis and small interfering RNA. On the other hand, the Th2 cytokine, IL-4, did not demonstrate any direct action whatsoever on BH expression. However, IL-4 down-regulated MZF-1 and Sp-1 in cultured keratinocytes and thus it is suggested that it acts as a suppressor of BH regulation. Finally, expression of BH was investigated in the skin of patients suffering from atopic dermatitis (AD). Since the activity and expression of BH decreased considerably in AD lesional skin, a defect in the filaggrin degradation pathway was suggested to be present in AD. As has been described above, the inventors of the present invention found that transcription of BH is most likely regulated during both differentiation and inflammation. Thus, as a result of investigating the bleomycin hydrolase production promoting activity of various pharmaceutical agents and herbal medicines, the inventors of the present invention found that certain drugs and herbal medicines have that activity, thereby leading to completion of the present invention.

The subject-matter of the present invention is defined in the claim.

### Effects of the Invention

The present invention enables provision of a novel NMF production promotor and a dry skin remedy.

### BRIEF DESCRIPTION OF THE DRAWINGS

FIG. 1 is a western blot diagram indicating the relationship between the amounts of bleomycin hydrolase in human skin extracts obtained by tape stripping and the number of times tape stripping is performed.
FIG. 2 is a western blot diagram indicating the relationship between the amounts of bleomycin hydrolase in human skin extracts and dry skin, wherein T and A indicate samples derived from subjects not having dry skin, N indicates a sample derived from a subject having somewhat dry skin, and M indicates a sample derived from a subject having dry skin.
FIG. 3 is a graph indicating the relationship between the amounts of bleomycin hydrolase present in a horny layer extract obtained from the arms of human subjects and the enzyme activity thereof, wherein the numbers indicated on the horizontal axis represent subject identification numbers.
FIG. 4 indicates values obtained by first order approximation using the least-squares method for the relationship between the amounts of bleomycin hydrolase obtained in FIG. 3 and the activity thereof.
FIG. 5 indicates the results of statistical analyses relating to bleomycin hydrolase present in a corny layer extract obtained from the arm of a human subject and skin parameters (A: free amino acids, B: activity, C: transepidermal water loss (TEWL)), wherein "BH low" indicates an amount of bleomycin hydrolase of less than 10 and activity of less than 1.5 (nmol/min/ml), and "BH high" indicates an amount of bleomycin hydrolase of equal to or greater than 10 and activity of equal to or greater than 1.5 (nmol/min/ml).
FIG. 6 is a flow chart of a questionnaire for classifying skin.
FIG. 7 indicates the results of measuring skin parameters of the corny layer of subjects classified according to the flow chart of FIG. 6.
FIG. 8 indicates tissue staining diagrams showing localization of bleomycin hydrolase and filaggrin in normal skin.
FIG. 9 indicates tissue staining diagrams showing localization of bleomycin hydrolase and filaggrin in the skin of atopic dermatitis patients.
FIG. 10 is a graph indicating the relationship between keratinocyte differentiation and expression levels of bleomycin hydrolase using quantitative PCR, wherein values on the vertical axis represent relative amounts in the case of assigning a value of 1 for the expression level after reaching 80% confluence.
FIG. 11 is a schematic diagram showing the 5'-flanking region of the gene that encodes bleomycin hydrolase.
FIG. 12 is a graph indicating the results of a luciferase assay of BH promoter using human epidermal keratinocytes.
FIG. 13 is a graph indicating the relationship between expression of transcription factors Sp-1, MZF-1 and GATA-1 and UV irradiation.
FIG. 14 is a graph indicating the relationship between bleomycin hydrolase levels in normal human epidermal keratinocytes and protease expression.
FIG. 15 indicates primers used to produce mutants having consecutive 5'-defects in the 5'-flanking region of BH by PCR.
FIG. 16 indicates primers used to analyze the transcription levels of BH and related factors by quantitative real-time RT-PCR.
FIG. 17 indicates probes used to analyze electrophoretic mobility shift.
FIG. 18(A) indicates a schematic drawing of the 5'-flanking region of human BH, wherein the presumed transcription factor binding site in the 5'-flanking region was determined by a search using the Genome Net Motif Program. FIG. 18(B) indicates the BH promoter region as determined by deletion analysis. FIG. 18(C) indicates the nucleotide sequence of the -216/-1 region containing the minimal promoter sequence of BH and the presumed transcription factor binding sites, wherein the presumed transcription factor binding sites are underlined.

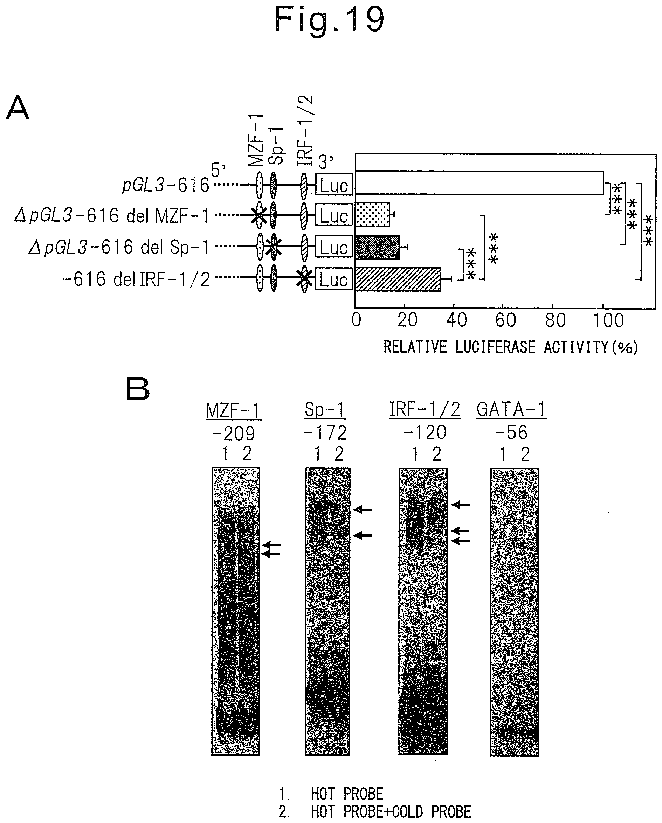

FIG. 19(A) indicates the results of determining properties of transcription factor binding sites in BH promoter by site-specific mutagenesis consisting of a schematic diagram of a deletion construct of the presumed transcription factor binding site that indicates the luciferase activity thereof in cultured keratinocytes, wherein site-specific mutagenesis was carried out using a construct that spans the nucleotide sequence of the - 616/+1 region. FIG. 19(B) indicates the binding of MZF-1, Sp-1, GATA-1 or IRF-1/2 to a cis-acting element of BH promoter, wherein an experiment was carried out in the form of an electrophoretic mobility shift assay (EMSA) using nuclear extracts obtained from cultured keratinocytes and biotinylated double-stranded oligonucleotide probes containing presumed transcription binding site MZF-1, Sp-1, GATA-1 or IRF-1/2, with lane 1 indicating the binding profile of biotinylated probe in the nuclear extract, and lane 2 indicating the binding profile of biotinylated probe following competitive binding with a non-labeled probe present in excess in an amount twice that of the biotinylated probe.

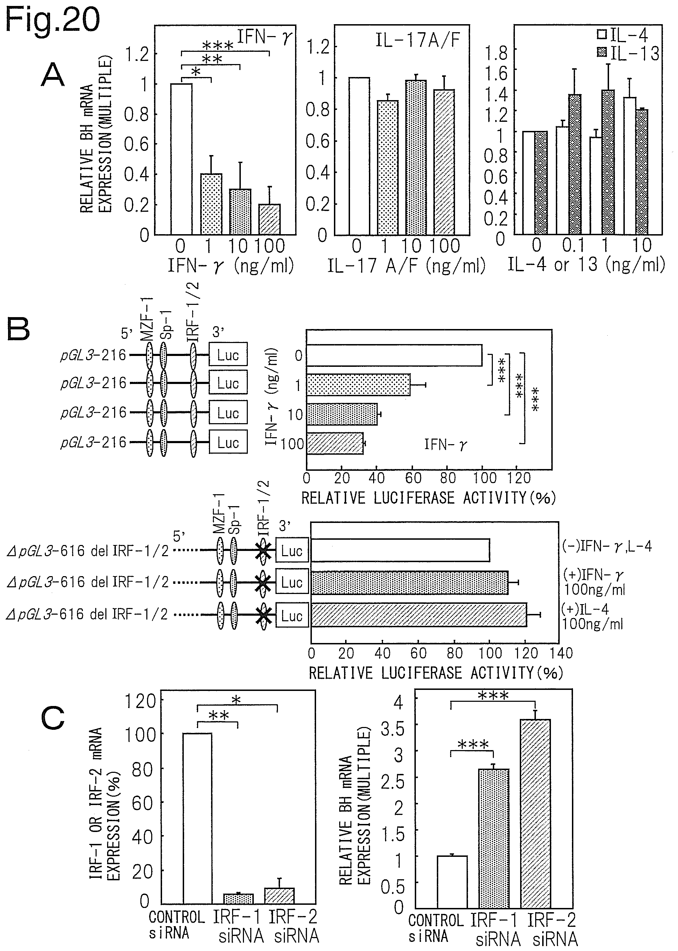

FIG. 20(A) indicates the results of a real-time RT-PCR analysis of BH expression showing the effects of Th1, Th2 and Th17 cytokines on expression of BH gene. FIG. 20(B) indicates the results of a mutation analysis of the IRF-1/2 binding site showing BH promoter activity in cultured keratinocytes in the presence of IFN-γ as determined by transfecting keratinocytes with pGL3-216 containing the intact IRF-1/2 binding sites of the BH promoter region followed by treating with IFN-γ for 24 hours (upper panel), and by transfecting keratinocytes with ΔpGL3-616 (IRF-1/2 deletion mutant) followed by treating for 24 hours in the presence or absence of IFN-γ or IL-4 at a concentration of 10 mg/ml (lower panel). FIG. 20(C) indicates the results of measuring expression of IRF-1 and IRF-2 genes using small interfering RNA (siRNA) for determining whether or not IRF-1/2 is an essential mediator for IFN-γ-induced down-regulation by transfecting keratinocytes with siRNA of IRF-1 or IRF-2 (40 nM) followed by culturing for 24 hours, treating with 10 ng/ml IFN-γ and further culturing for 24 hours followed by isolating the RNA, with the panel on the right side indicating the silencing effects of IRF-1 and IRF-2.
FIG. 21(A) indicates the results of an analysis of the expression of BH, calpain-1 and presumed transcription factors in proliferating cells or differentiated cells by real-time PCR for investigating regulation of transcription in the epidermis. FIG. 21(B) indicates the results of an analysis of the expression patterns of transcription factors MZF-1, Sp-1, GATA-1, IRF-1 and IRF-2 in cultured keratinocytes.
FIG. 22(A) indicates the effects of IFN-γ on expression of presumed transcription factors IRF-1 and IRF-2. FIG. 22(B) indicates the effects of IL-4 on expression of presumed transcription factors IRF-1, IRF-2, MZF-1 and Sp-1.
FIG. 23(A) indicates simultaneous localization of BH and filaggrin in the granular layer as indicated by double staining with anti-BH antibody and anti-filaggrin antibody in normal epidermis. FIG. 23(B) indicates the BH activities of extracts from lesional skin and non-lesional skin of an AD patient.
FIG. 24 indicates the promoting effects of various herbal medicines and drugs on production of bleomycin hydrolase.

### BEST MODE FOR CARRYING OUT THE INVENTION

Bleomycin hydrolase is a cytoplasmic cysteine peptide hydrolytic enzyme having a molecular weight of 250 kDa to 280 kDa (hexamer), and its initially known function was metabolic deactivation of the glycopeptide bleomycin, which is frequently used in cancer combination chemotherapy. Bleomycin hydrolase contains the characteristic active site residues of the papain superfamily of cysteine proteases, and its encoding gene is present at genetic locus 17q11.2 in humans (Takeda, et al., J. Biochem., 119, 29-36, 1996). It is present in all tissues and although it is known to also be present in skin (Kamata, et al., J. Biochem., 141, 69-76, 2007), its relationship with filaggrin was completely unknown until revealed by the inventors of the present invention.

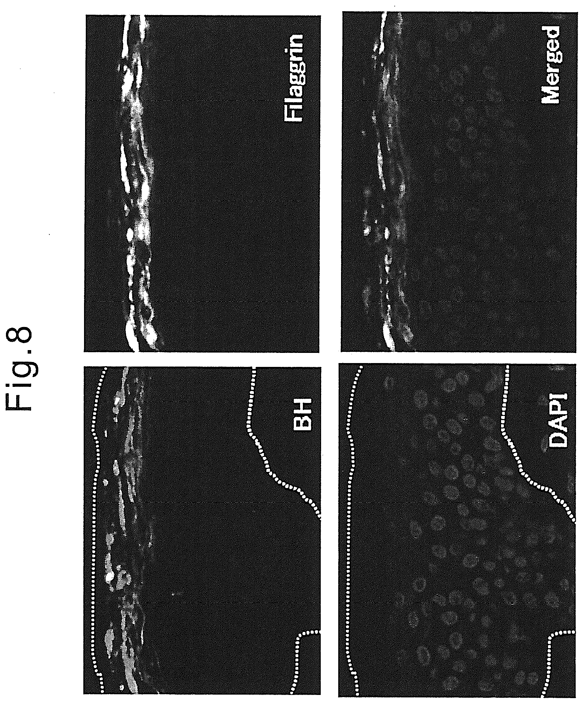

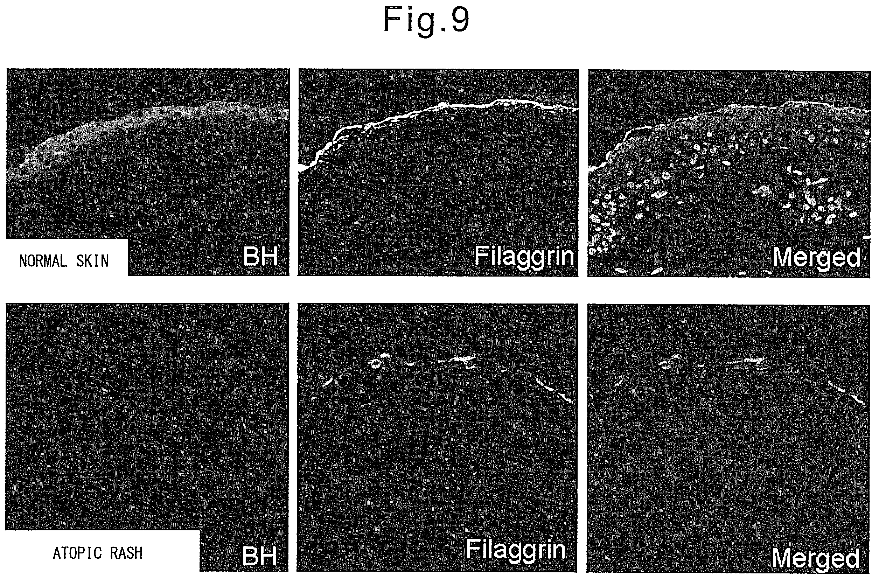

Based on the results of tissue staining, bleomycin hydrolase was determined to be expressed at high levels in the upper layer of the epidermis in normal skin in the same manner as filaggrin (FIG. 8). On the other hand, in patients with atopic dermatitis, the expression of this enzyme as well as filaggrin decreases at locations of atopic rash (FIG. 9). This strongly suggests that the cause of atopic dermatitis is not an abnormality of the profilaggrin gene, but rather an abnormality of the enzyme system responsible for its degradation. In addition, bleomycin hydrolase activity is significantly lower not only in the lesional areas of the skin of atopic dermatitis patients, but also in non-lesional areas as well (data not shown).

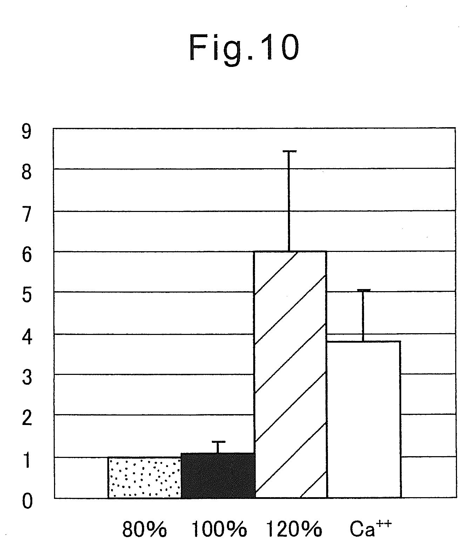

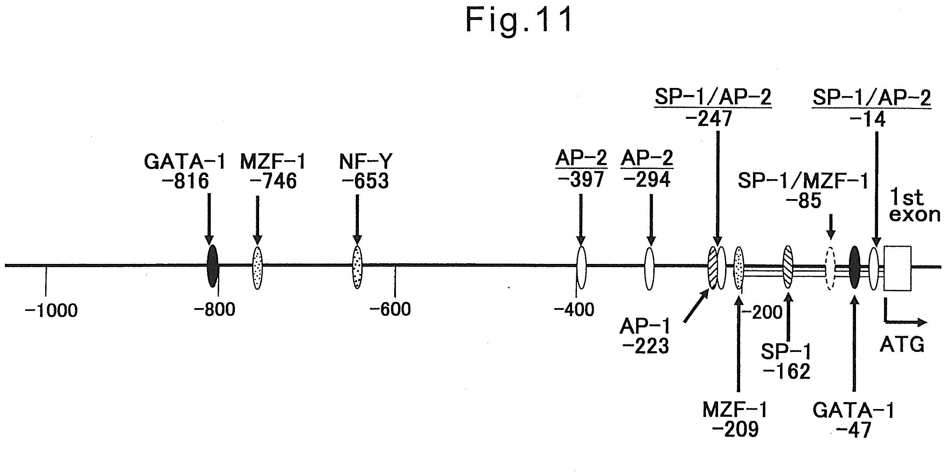

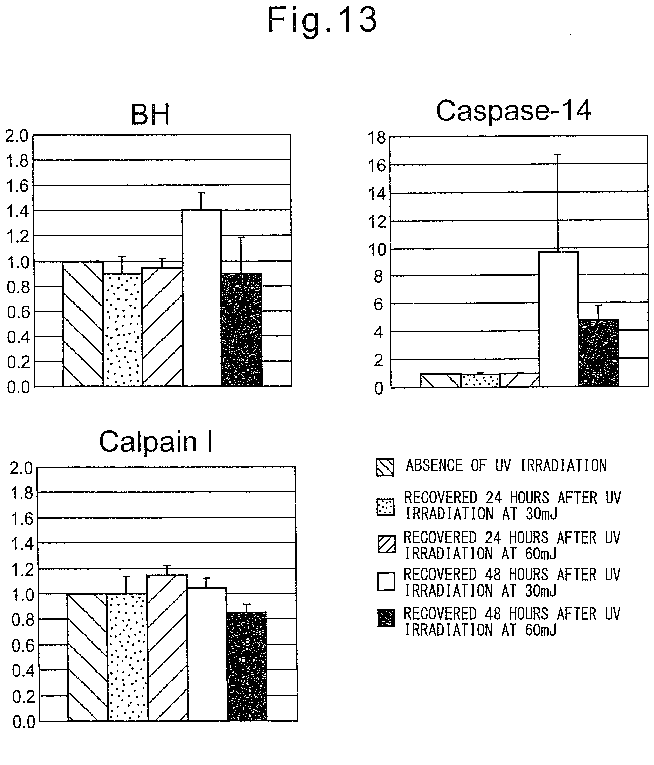

Moreover, as a result of examining fluctuations in expression levels of bleomycin hydrolase using cultured keratinocytes, in contrast to this enzyme being hardly expressed at all in undifferentiated keratinocytes, it was determined to be highly expressed in keratinocytes that have reached confluence and in which differentiation has progressed, and although hardly expressed at all in basal cells, was determined to be highly expressed after migrating to epidermal cells after differentiation had progressed (FIG. 10). This result supports the results of the aforementioned cell staining. The 5'-flanking region of the gene that encodes bleomycin hydrolase, and particularly, the transcription regulatory region and transcription factors binding to this region, are shown in FIG. 11. A region extending at least 216 bp downstream from the coding sequence of bleomycin hydrolase is required to be included in order to express this enzyme. The expression of bleomycin hydrolase is thought to be especially promoted by promoting the binding activity of those transcription factors among the transcription factors described in FIG. 11 of IRF-1, IRF-2, MZF-1, SP-1 and GATA-1 contained in this region. In fact, when the expression of bleomycin hydrolase is promoted by ultraviolet (UV) irradiation (data not shown), a correlation is observed between promotion of expression of MZF-1 and GATA-1 and the intensity and duration of UV irradiation (FIG. 13).

Promotion of the production of bleomycin hydrolase is also affected by cytokines. For example, interleukin-4 (IL-4), which is a type of Th2 cytokine known to be involved in atopic dermatitis, down-regulates the expression of bleomycin hydrolase. This supports the low expression levels of bleomycin hydrolase observed in the skin of atopic dermatitis patients. On the other hand, interferon-γ, which is a typical representative of a Th1 cytokine having the ability to inhibit IgE production in contrast to IL-4, significantly increases the expression of bleomycin hydrolase. In addition, a Th2 cytokine that is also a typical example of an inflammatory cytokine, tumor necrosis factor alpha (TNFα) also significantly increases expression of this enzyme. In addition to these substances, expression and/or activity of bleomycin hydrolase is also increased by UV irradiation. Although the results thereof are not shown, on the surface of the body as well, the activity of bleomycin hydrolase in the skin of the cheeks susceptible to UV irradiation has been confirmed to be increased by UV irradiation.

Although chestnut rose extract, angelica root extract, cork tree bark extract, lamium album extract and rosemary extract are used in external skin preparations, none of these extracts were known to have bleomycin hydrolase production promoting effects, NMF production promoting effects or effects for improving dry skin. For example, chestnut rose extract is only known to have a ceramide synthesis promoting effect (Japanese Unexamined Patent Publication No. 2006-111560) and a collagenase inhibitory effect (Japanese Unexamined Patent Publication No. 2006-241148). Although benzenesulfonyl GABA (benzenesulfonyl γ-aminobutyric acid) and erythritol are also similarly used in external skin preparations, neither of these drugs were known to have bleomycin hydrolase production promoting effects or NMF production promoting effects.

The aforementioned extracts can be obtained in accordance with ordinary methods, and for example, can be obtained by immersing or refluxing a portion or all of a source plant with an extraction solvent either at normal temperature or while heating, followed by filtration and concentration. The extracted site may be dried prior to solvent extraction. Any solvent can be used for the extraction solvent provided it is a solvent that is normally used for extraction, and examples of thereof include organic solvents in the manner of alcohols such as methanol, ethanol, propylene glycol, 1,3-butylene glycol or glycerin, water-containing alcohols, chloroform, dichloroethane, carbon tetrachloride, acetone, ethyl acetate, hexane, as well as aqueous solvents such as water, physiological saline, phosphate buffer or borate buffer, and these can be used either alone or in combination. One type or two or more types selected from the group consisting of water, methanol, ethanol and 1,3-butylene glycol are preferably used as solvent.

Extract obtained by extracting with solvent in the manner described above can be used directly or after concentrating by freeze-drying and the like, or as necessary, may be removed of impurities using an adsorption method such as an ion exchange resin, or can be used after adsorbing with a porous polymer column (such as the Amberlite XAD-2 column), eluting with a desired solvent and then concentrating.

Extracts such as chestnut rose extract, angelica root extract, cork tree bark extract, lamium album extract and rosemary extract, and drugs such as benzenesulfonyl GABA and erythritol, demonstrate an action that concentration-dependently promotes production of bleomycin hydrolase. Thus, from this viewpoint, the incorporated amounts of extracts such as chestnut rose extract, angelica root extract, cork tree bark extract, lamium album extract and/or rosemary extract in the bleomycin hydrolase production promotor of the present disclosure is 0.0001% by weight to 20.0% by weight, preferably 0.0001% by weight to 10.0% by weight, and more preferably 0.001% by weight to 1% by weight as the dry weight thereof based on the total weight of the agent. The incorporated amount of a drugs such as benzenesulfonyl GABA and/or erythritol is 0.0001 mmol to 20.0 mmol, preferably 0.0001 mmol to 10.0 mmol and more preferably 0.001 mmol to 1 mmol as the dry weight thereof based on the total weight of the agent.

The bleomycin hydrolase production promotor according to the present disclosure can be produced in accordance with ordinary methods. In addition, although it can also be prepared by using one type or two or more types of the aforementioned extracts and drugs as components of the bleomycin hydrolase production promotor, components normally used in external skin preparations such as cosmetics or pharmaceuticals containing quasi drugs are also suitably incorporated as necessary, examples of which include oils, surfactants, powders, colorants, water, alcohols, thickeners, chelating agents, silicones, antioxidants, ultraviolet absorbers, moisturizers, fragrances, various medicinal ingredients, antiseptics, pH regulators and neutralizers.

There are no particular limitations on the dosage form of the bleomycin hydrolase production promotor of the present disclosure, and it can have an any arbitrary form such as a solution system, solubilized system, emulsified system, powder dispersed system, water-oil double-layered system, water-oil-powder triple-layered system, ointment, gel or aerosol. In addition, there are also no particular limitations on the form of use, and can be used in any arbitrary form such as a beauty wash, milky lotion, cream, essence, jelly, gel, ointment, facial pack, mask or foundation.

Since the bleomycin hydrolase production promotor of the present disclosure is applied to the skin, it can be used in beauty treatments for preventing and/or improving dry skin. There are no particular limitations on the manner of use or dosage of the bleomycin hydrolase production promotor of the present disclosure when used in such beauty treatments, and although the manner of use and dosage is suitably determined according to the drug form or status of skin wrinkling to be treated, typically a suitable amount of from 0.1 ml to 1 ml per cm² is rubbed directly into the skin or that suitable amount is impregnated into a piece of gauze and then applied to the skin several times per day, and for example, 1 to 5 times per day.

The following provides a more detailed explanation of the present disclosure. Furthermore, the present invention is defined by the claim.

### Examples

The following materials were used in the present experiments.

Calpain-1 was purchased from EMD Biosciences, Inc. Bleomycin hydrolase was prepared from the horny layer of human epidermis in accordance with Non-Patent Document 5. Human IL-4 and IFN-γ were purchased from Peprotech EC (London, England). Human IL-13 and IL-17A/F were manufactured by R&D Systems Inc. (Minneapolis, MN). Citrulline 4-methylcoumaryl-7-amide (Cit-MCA) was acquired from Bachem Bioscience AG (Bubendorf, Switzerland). Reagent grade chemicals were used for all other chemical substances used.

### Keratinocyte Culturing

Normal human epidermal keratinocytes derived from neonatal epidermis (Kurabo Industries, Ltd., Osaka, Japan) were cultured in EpiLife medium (Cascade Biologics, Inc., Portland, OR) containing low-concentration (0.03 mM) calcium and HKGS Growth Supplement (Cascade Biologics, Inc.). All cells were incubated at 37°C in the presence of 5% CO₂ and were used within 4 passages of subculturing. Cells were collected at 70% confluence, 100% confluence, 2 days after reaching confluence, and 2 days after reaching confluence in 2 mM calcium.

### Experiment 1

Bleomycin hydrolase is thought to act in the final stage of NMF production. In this case, there is the possibility of expression of this enzyme being decreased in dry skin. In this experiment, a study was conducted as to whether or not decreases in expression and/or activity of bleomycin hydrolase in skin are related to dry skin.

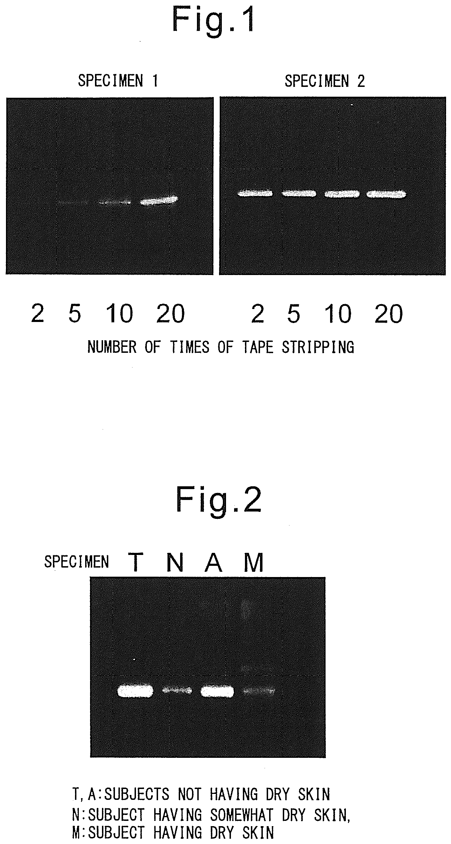

Skin horny layer samples were collected by tape stripping consisting of affixing clear adhesive tape (CelloTape™, Nichiban Co., Ltd.) to a skin surface on the arm followed by peeling off the tape. The tape having the skin horny layer adhered thereto was then cut into pieces, immersed in extraction buffer (0.1 M Tris-HCl (pH 8.0), 0.14 M NaCl, 0.1% Tween-20, 1 ml) and then subjected to ultrasonic treatment (20 sec × 4 rounds) to prepare horny layer extracts. These extracts were then subjected to western blotting. The anti-bleomycin hydrolase (BH) antibody used was produced according to the method of Kamata, et al. (Journal of Biological Chemistry 2009). More specifically, after subjecting the horny layer extract to electrophoresis, it was transferred to Immobilon-P (Millipore Corp.), and after washing the transferred film, was allowed to react with anti-BH antibody for 1 hour at room temperature. After removing the antibody by additional washing, the extract was reacted with HRP-bound secondary antibody. After washing, the BH protein band illuminated with the ECL Plus Western Blotting Kit (GE Healthcare Inc.) was baked onto X-ray film and expression levels were estimated based on the degree of shading of the band. The results are shown in FIGS. 1 and 2.

In FIG. 1, Specimen 1 indicates a skin horny layer sample of a person personally thought to have dry skin, while Specimen 2 is a skin horny layer sample of a healthy student thought not to have dry skin. In addition, Specimens T and A in FIG. 2 are from subjects not having dry skin, Specimen N is from a subject having somewhat dry skin, and Specimen M is from a subject having dry skin. Although the expression level of bleomycin hydrolase in Specimen 1 was low, the expression level of that enzyme in Specimen 2 was high. On the basis of this result, Specimens 1 and 2 can be understood to be derived from dry skin and moist skin, respectively. In addition, based on the results from using Specimen 1, in the case of dry skin, it appears that the amount of bleomycin hydrolase decreases at sites closer to the epidermal surface where NMF production occurs. In FIG. 2, Specimens T and A indicate western blots of extracts obtained from specimens not particularly aware of having dry skin, while Specimens N and M indicate western blots of extracts obtained from specimens strongly aware of having dry skin.

### Experiment 2

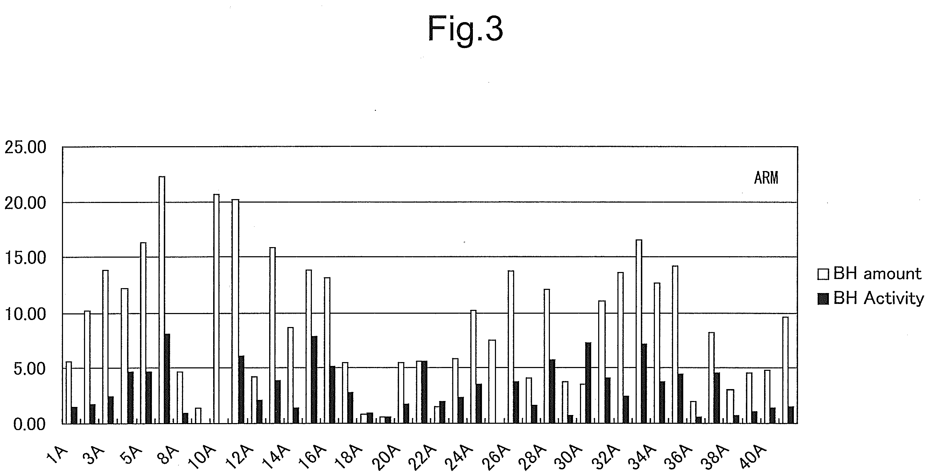

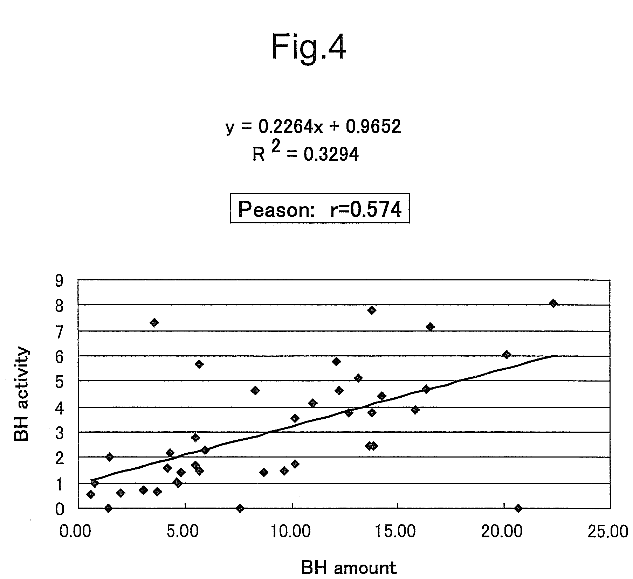

In this experiment, a study was conducted of individual differences in the amount and activity of bleomycin hydrolase in human skin along with an examination of the relationship between those amounts and activity. Horny layer extracts were prepared from skin of the arms of 40 female students age 20 to 25 in accordance with the method described in Experiment 1. The amount and activity of bleomycin hydrolase present in the extracts were measured in accordance with the method of Kamata, et al. (J. Biol. Chem., Vol. 284, Issue 19, 12829-12836, May 8, 2009). Expression levels were evaluated by western blotting, while enzyme activity was evaluated for the aminopeptidase activity of this enzyme by measuring the degraded amount of a fluorescent substrate, Cit-β-NA. The results are shown in FIG. 3, while a correlation diagram is shown in FIG. 4. As is clear from the results shown in FIG. 4, a correlation exists between the amount of bleomycin hydrolase and the activity thereof.

Continuing, a statistical analysis was conducted relating to bleomycin hydrolase and various skin parameters for the aforementioned horny layer extracts. In this experiment, the horny layer extracts of 40 subjects were classified into the following two types. After having digitized the amounts of bleomycin hydrolase determined on the basis of western blotting with a densitometer, those extracts in which the amount of bleomycin hydrolase is less than 10, in the case of indicating based on an arbitrary unit of 1, and enzyme activity is less than 1.5 nmol/min/ml were classified as having a low amount of bleomycin hydrolase protein and having low enzyme activity (BH low), while all other extracts were classified as having a high amount of protein and high enzyme activity (BH high).

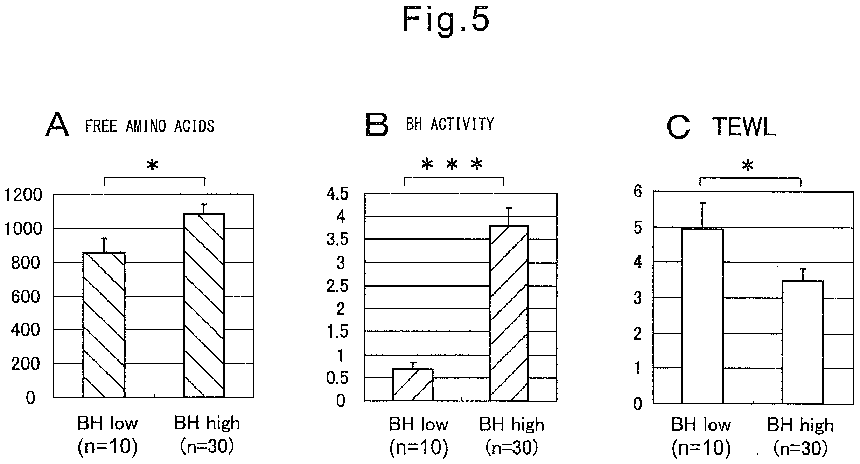

Free amino acids were measured in accordance with the method of Kamata, et al. (J. Biol. Chem., Vol. 284, Issue 19, 12829-12836, May 8, 2009). More specifically, filaggrin peptide degraded with calpain-1 was allowed to react with each extract followed by measurement of the amount of free amino acids by quantifying amino groups using fluorescamine. The results of measuring free amino acids are shown in FIG. 5A. Units on the vertical axis in FIG. 5A indicate the total amount of free amino acids (nmol) in 3 ml of measurement sample.

Bleomycin hydrolase activity was evaluated by measuring the amount of a fluorescent substrate, Cit-β-Na degraded by the aminopeptidase of this enzyme as previously described. The results of measuring bleomycin hydrolase activity are shown in FIG. 5B. Units on the vertical axis in FIG. 5B indicate the degraded amount of Cit-β-Na (nmol/min/ml).

Transepidermal water loss (TEWL) of the skin of the aforementioned students was measured using a Vapometer (Delfin Technologies, Ltd., Finland) and expressed as g/m²/h. Results of measuring TEWL are shown in FIG. 5C.

As shown in FIG. 5C, there was a significant difference in horny layer moisture content between the low bleomycin hydrolase activity group (less than 2.5 U) and the high group. Moreover, there were few free amino acids and TEWL was high in a group having both low enzyme amounts and activity (FIGS. 5A and 5C).

Although the data is not shown, a significant difference in the amounts of NMF and urocanic acid was present between free amino acid low (less than 1000) and high groups, and a significant difference in the amount of urocanic was present between NMF low (less than 0.8) and high groups. In addition, significant differences in NMF, lactic acid and urea were present between the TEWL low (less than 2.5) and high groups. When considering that urocanic acid is produced from histidine contained in large amounts in filaggrin, bleomycin hydrolase can be understood to be important in the degradation of filaggrin.

Based on the results of this experiment, in cases in which the absolute amount of bleomycin hydrolase is low, both the amount of free amino acids and barrier function can be understood to decrease significantly. Although the data is not shown, even in the case of using a cheek-derived horny layer extract, a proportional relationship was confirmed between the amount of bleomycin hydrolase and the skin's barrier function.

### Experiment 3

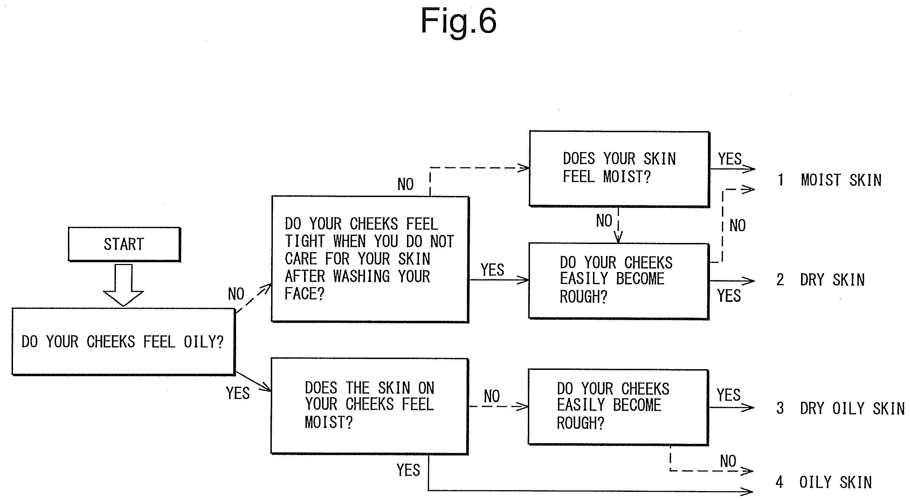

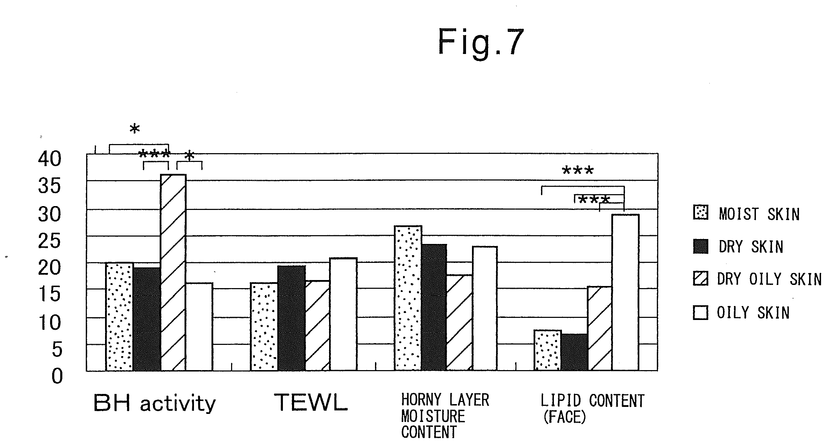

In this experiment, a survey was conducted among the aforementioned female students based on the flow chart shown in FIG. 6, and the skin of each student was classified into one of four categories consisting of moist skin, dry skin, dry oily skin or oily skin. The survey results and correlations with the results for skin parameters measured in the aforementioned Experiment 2 are shown in FIG. 7. Based on the data shown in FIG. 7, bleomycin hydrolase activity was significantly higher in those students classified as having oily dry skin.

### Experiment 4

In this experiment, a study was conducted for the amount of bleomycin hydrolase present in skin and localization of filaggrin.

### Immunohistochemical Staining

Immunohistochemical staining was carried out according to the method of Kamata, et al. (J. Biol. Chem., Vol. 284, Issue 19, 12829-12836, May 8, 2009). Samples consisted of frozen sections of human skin having a thickness of 5 µm, and anti-rat BH IgG were used. More specifically, human skin specimens were obtained from patients suffering from atopic dermatitis being treated at the Tokyo Medical University after obtaining their informed consent. This study was approved by the Institutional Review Board of Tokyo Medical University relating to Human Ethics and by a special subcommittee of Shiseido Co., Ltd.

Sections of human atopic dermatitis (lesional skin and non-lesional skin) and normal skin were incubated with anti-rat BH IgG and anti-human filaggrin IgG for 1 hour at room temperature followed by washing with PBS and further incubating with fluorescent-bound secondary antibody, Alexa Fluor 555 or 488 (Molecular Probes Inc., Eugene, OR). DAPI (4',6'-diamidino-2-phenylindole, Molecular Probes Inc.) was used to visualize nuclei.

The results for immunostaining normal skin are shown in FIG. 8, while the results of comparing skin from a healthy individual (normal skin) with skin from an atopic dermatitis patient (atopic rash) are shown in FIG. 9. As shown in FIG. 8, bleomycin hydrolase is highly expressed in the upper layer of the epidermis and demonstrated the same localization as filaggrin. On the other hand, at locations of atopic rash, expression of bleomycin hydrolase and filaggrin was lower in comparison with that of normal skin (FIG. 9).

### Quantitative PCR

The expression level of bleomycin hydrolase in keratinocytes was measured by quantitative PCR according to the following method using Light Cycler 480 (Roche Diagnostics GmbH, Mannheim, Germany). Light Cycler FastStart DNA Master CYBR Green I was used for the reagent. 0.6 µl aliquots of each of the following bleomycin hydrolase primers and 6.8 µl of water were added to 10 µl of SYBR Green I Master Mix followed by bringing to a total volume of 20 µl and carrying out PCR for 45 cycles consisting of 15 seconds at 95°C, 20 seconds at 55°C and 20 seconds at 72°C. The results obtained were corrected by comparing with the results for a housekeeping gene, G3PDH.
Forward primer: TGTGGTTTGGCTGTGATGTT (SEQ ID NO: 1)
Reverse primer: GCACCATCCTGATCATCCTT (SEQ ID NO: 2)

The results of the aforementioned quantitative PCR are shown in FIG. 10. As shown in FIG. 10, bleomycin hydrolase was more highly expressed in keratinocytes that had reached confluence, namely differentiated keratinocytes, than keratinocytes at 80% confluence, namely undifferentiated keratinocytes. In other words, according to the results of this experiment, this enzyme can be understood to not be expressed that much in basal cells prior to differentiation. These results of quantitative PCR support the results of the aforementioned tissue staining.

### Experiment 5

### 1) Luciferase Assay of BH Promoter Using Human Epidermal Keratinocytes

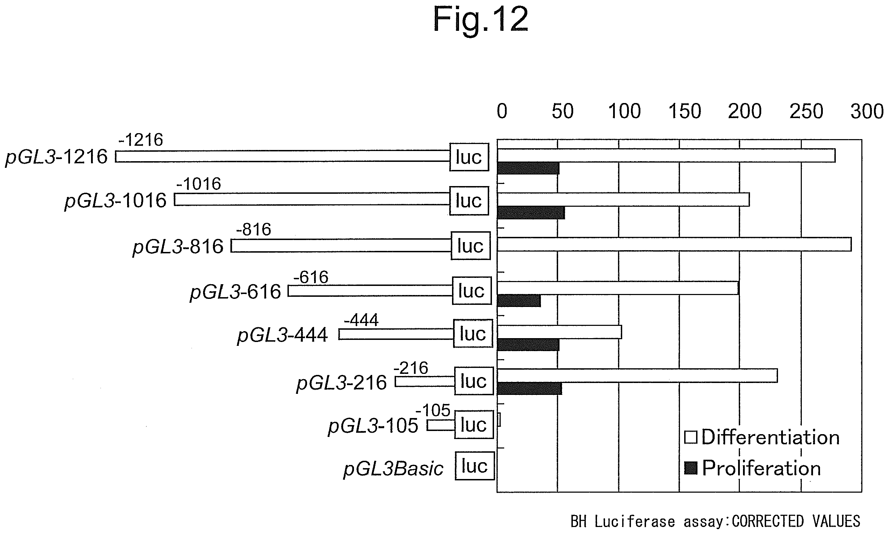

Lysis buffer (200 µl) was added to keratinocytes in the proliferation stage (roughly 80% confluence) or following differentiation (after reaching confluence, obtained by exposing to air, adding 2 mM calcium and continuing to culture for 2 more days) to lyse the cells. The Bright-Glo Luciferase Assay System (Promega Co., Madison, WI, USA) was used for measurement. 20 µl of sample were transferred to a prescribed tube and measured using the Auto Lumat Plus (LB953, Berthold GmbH & Co. KG, Bad Wilbad, Germany). Based on the results shown in FIG. 12, it was determined that a region extending at least 216 bp downstream from the coding sequence of bleomycin hydrolase must be present in the aforementioned transcription regulatory region in order to express this enzyme.

### 2) UV Irradiation of Normal Human Epidermal Keratinocytes (NHEK)

RNA was recovered by a prescribed method 3 hours, 24 hours and 48 hours after irradiating with UVB at 30 mJ or 60 mJ (Torex F120S-E-30/DMR, 20 W, Toshiba Medical Supply Co., Ltd.), and mRNA expression levels of bleomycin hydrolase and calpain were measured by quantitative PCR. As a result of these measurements, the highest level of bleomycin hydrolase mRNA was expressed by the sample recovered 48 hours after irradiating at 30 mJ (FIG. 13).

### 3) Effect of Cytokines on Bleomycin Expression

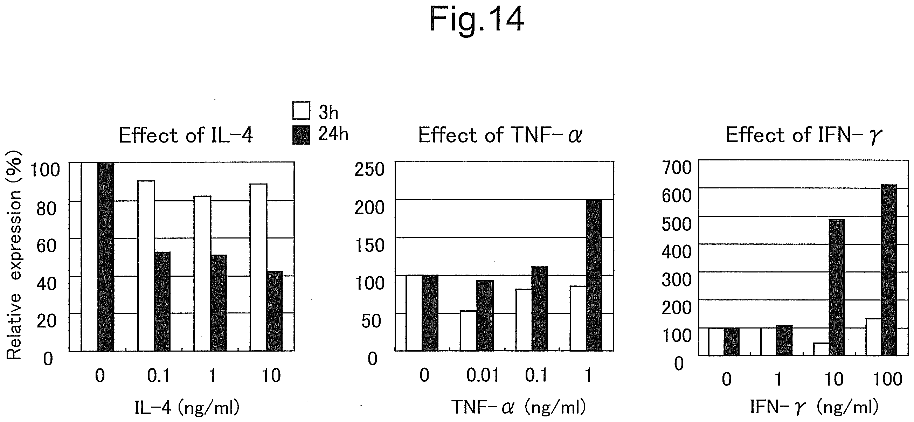

IL-4 (final concentration: 0.1, 1.0 or 10 ng/ml), TNFα (final concentration: 0.1, 1.0 or 10 ng/ml) and IFNγ (final concentration: 1.0, 10 or 100 ng/ml) were respectively added to cultured keratinocytes in the proliferation stage, and after incubating for 24 hours, RNA was collected using Isogen. Expression of bleomycin hydrolase mRNA was measured by quantitative PCR. Those results are shown in FIG. 14. Based on the results shown in FIG. 14, a type of cytokine, interleukin-4 (IL-4) can be understood to down-regulate expression of bleomycin hydrolase.

### Experiment 6

### Characterization of Human BH Gene

### 1) Cloning of BH 5'-Flanking Region

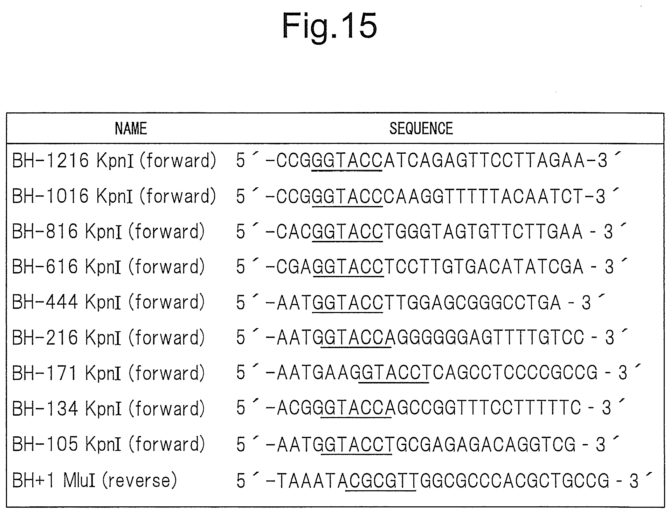

The 5'-flanking region was amplified based on the nucleotide sequence of human BH gene using the Genome Walker Kit (Clontech Laboratories, Inc., Mountain View, CA) in accordance with the instructions provided by the manufacturer and using Gene-Specific Primer 1 (GSP1): 5'-tccctcgagtctgtatcagagcagctaca-3' (SEQ ID NO: 3) and Gene-Specific Primer 2 (GSP-2): 5'-tgaacacgcgtccgagctgctcatggcg-3' (SEQ ID NO: 4). In brief, primary PCR was carried out using GSP1 and an Adapter Primer (AP) 1 according to the two-step PCR protocol recommended by the manufacturer (consisting of 7 cycles at 94°C for 25 seconds and 72°C for 4 minutes, followed by 32 cycles of 94°C for 25 seconds and 67°C for 4 minutes, and finally extension at 67°C for 4 minutes) using Ex Taq DNA Polymerase (Takara Corp., Shiga, Japan) in the presence of 5% dimethylsulfoxide. Next, the primary PCR mixture was diluted and then used as a template for secondary PCR amplification using GSP2 and AP2. Secondary PCR was carried out in the same manner as primary PCR with the exception of initially carrying out 5 cycles instead of 7 cycles, and subsequently carrying out 20 cycles instead of 32 cycles. Consecutive 5'-deletion mutant strains of the 5'-flanking region of BH were produced by PCR using the primers listed in FIG. 15. Following amplification, all PCR products were cloned into pGEM-T Easy Vector (Promega Co., Madison, WI) and then subjected to sequencing using the ABI Prism 310 Genetic Analyzer (Applied Biosystems Inc., Foster City, CA) .

In order to construct a reporter plasmid pGL3-1216/+1, PCR was carried out under the conditions of 30 cycles of initial denaturation consisting of 4 minutes at 94°C, 30 seconds at 94°C, 1 minute at 60°C and 1 minute at 72°C, and finally extension for 4 minutes at 72°C, using pGEM-T-1216/+1 as template and a pair of specific BH primers containing restriction sites KpnI and MIuI (5'-ccgggtaccatcagagttccttagaa-3' (SEQ ID NO: 5) and 5'-taaatacgcgttggcgcccacgctgccg-3' (SEQ ID NO: 6)). The resulting PCR products were digested with KpnI and MIuI and cloned in pGL3-Basic Vector (Promega Co.). Furthermore, pGL3-Basic Vector contains firefly luciferase gene. All constructs were prepared using the Qiagen Plasmid Midi Kit (Qiagen GmbH, Dusseldorf, Germany).

### 2) Site-Specific Mutagenesis

Mutagenesis at MZF-1, Sp-1 and IRF-1/2 binding sites was carried out by using the Quick Change Site-Directed Mutagenesis Kit (Stratagene Corp., La Jolla, CA). In order to create a deletion mutation in Sp-1, primers were used consisting of 5'-ggaccccgtttcagcctccccgcc-3' (SEQ ID NO: 7) (forward primer of mutant SP-1 site) and 5'-ggcggggaggctgaaacggggtcc-3' (SEQ ID NO: 8) (reverse primer of mutant Sp-1 site). With respect to the MZF-1 mutation, primers were used consisting of 5'-gactcagcaacgcggttttgtccctccgc-3' (SEQ ID NO: 9) (forward primer of mutant MZF-1 site) and 5'-gcggagggacaaaaccgcgttgctgagtca-3' (SEQ ID NO: 10) (reverse primer of mutant MSF-1 site). With respect to the IRF-1/2 mutant, primers were used consisting of 5'-gccgccgagcctccggcgctcc-3' (SEQ ID NO: 11) (forward primer of mutant IRF-1/2 site) and 5'-ggagcgccggaggctcggcggc-3' (SEQ ID NO: 12) (reverse primer of mutant IRF-1/2 site).

### 3) Transfection and Measurement of Promoter Activity

Keratinocytes were cultured in a 12-well tissue culture plate at a density of 5 × 10⁴ cells/well followed by transfection with 1 µg aliquots of each construct using FuGene HD Transfection Reagent (Roche Diagnostics AG, Basel, Switzerland). In order to correct for transfection efficiency, all cells were simultaneously transfected with pGL4.74[hRluc-TK] vector (Promega Co.) containing sea pansy (Renilla) luciferase gene under the control of HSV-TK promoter. Unless specifically indicated otherwise, the cells were collected 24 hours after transfection and lysed using 250 µl of Passive Lysis Buffer (Promega Co.) per well. Luciferase activity was analyzed using the Dual Luciferase Reporter Assay System (Promega Co.) and Auto Lumat Plus Luminometer (Berthold Technologies GmbH, Bad Wilbad, Germany). Firefly luciferase activity was standardized for sea pansy luciferase activity. Three transfections were independently carried out for each construct and results were expressed as the mean value thereof.

### 4) Quantitative Real-Time RT-PCR Analysis

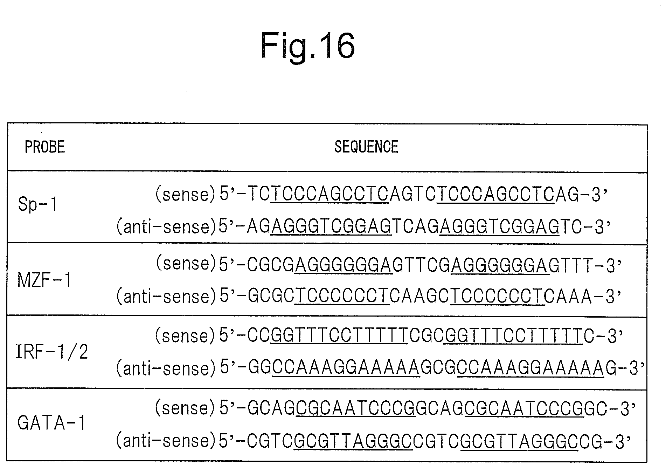

Transcription levels of BH and related factors were analyzed by quantitative real-time RT-PCR. Total RNA was extracted from cultured cells using Isogen (Nippon Gene Co., Ltd., Tokyo, Japan) in accordance with the instructions provided by the manufacturer. Reverse transcription of cDNA was carried out using superscript™ II (Invitrogen Corp., Carlsbad, CA). Real-time RT-PCR was carried out with the Light Cycler Raid Cycler System using the Light Cycler 480 SYBR Green I Master Mix (Roche Diagnostics GmbH) in accordance with the instructions provided by the manufacturer. Information relating to the primers used is shown in FIG. 16. Glyceraldehyde 3-phosphate dehydrogenase (GAPDH) was used as a housekeeping gene. Specificity of the amplified fragments was confirmed by quantitative analysis of melting curves by Light Cycler analytical software. The amounts of mRNA were standardized with respect to GAPDH mRNA, and finally indicated as a ratio to the mRNA of an untreated control.

### 5) siRNA-Based Suppression of IRF-1 and IRF-2

Cultured keratinocytes were transfected using 40 nM siIRF-1, siIRF-2 and siControl A (Santa Cruz Biotechnology Inc., Santa Cruz, CA) together with Lipofectamine RNAi Max (Invitrogen Corp., Carlsbad, CA) in accordance with the instructions provided by the manufacturer. After culturing the cells for 24 hours in antibiotic-free medium, total RNA was extracted and analyzed by real-time RT-PCR in the manner previously described.

### 6) Electrophoretic Mobility Shift Analysis (EMSA)

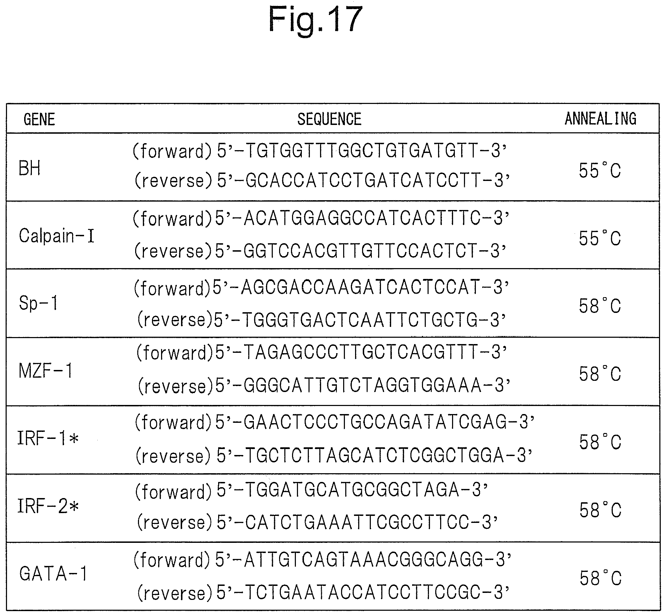

Double-stranded oligonucleotide probes were prepared by annealing a single-stranded biotinylated oligonucleotide and single-stranded non-labeled oligonucleotide (FIG. 17). Nuclear extraction and EMSA were carried out by using the Nuclear Extraction Kit and EMSA Gel Shift Kit (Panomics, Inc., Santa Clara, CA).

The nuclear extracts (4 µg) were incubated with 1× binding buffer, 1 µg of Polyd (I-C) and biotinylated probes (50 pmol) corresponding to the MZF-1, Sp-1, IRF-1/2 and GATA-1 binding sites for 30 minutes at 15°C. In order to conduct a competitive assay, a two-fold excess amount of unlabeled probe was added to the binding reaction prior to addition of biotinylated probe. These incubated mixtures were electrophoresed in 8% polyacrylamide gel together with 5x TBE buffer and then transferred to a Biodyne B Nylon Membrane (Pall Corp., Port Washington, NY). Bands were visualized using the chemiluminescence detection reagent provided in the EMSA Gel Shift Kit.

### 7) Results

### Isolation and Characterization of Human BH Gene Promoter

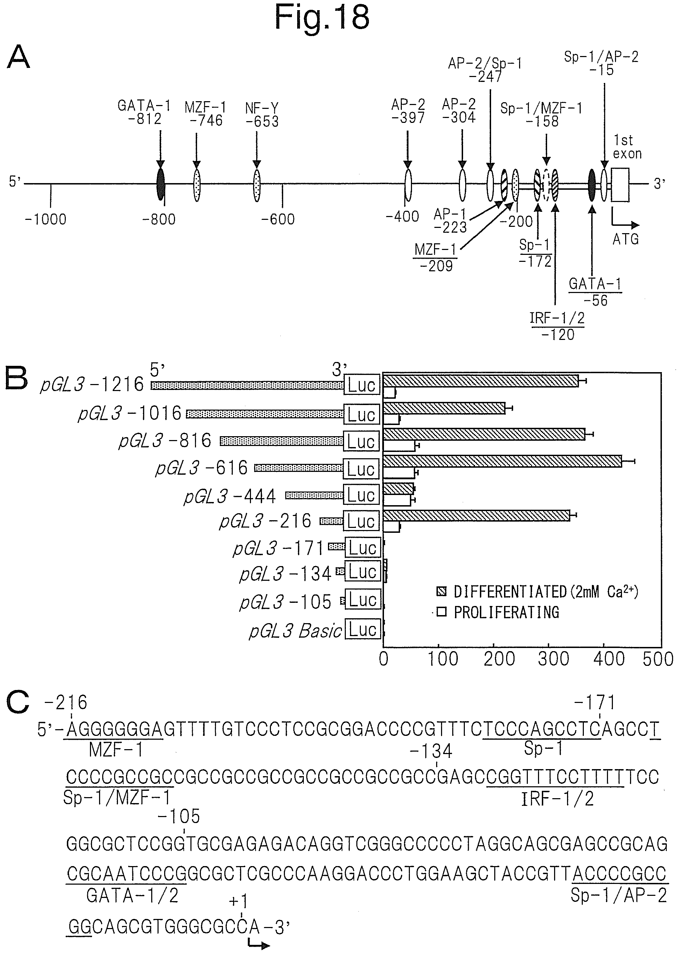

A large number of presumed transcription factor binding sites in the 5'-flanking region of human BH were identified by searching with the Genome Net Motif program (FIG. 18A). Since sequences closely coinciding with the consensus sequences recognized by MZF-1, Sp-1, IRF-1/2 and GATA-1/2 were present in the -216/+1 region close to the transcription initiation site in particular, these transcription factors were suggested to be involved in the regulation of BH promoter activity. A deletion analysis was carried out in order to more precisely determine the BH promoter region (FIG. 18B). The highest level of luciferase activity was detected in differentiated keratinocytes transfected with pGL3-816. However, the relative luciferase activity of the deletion plasmids remained high until deletion proceeded to pGL3-216. In this construct (indicated as pGL3-444), a plasmid containing the fragment -444/+1 demonstrated significantly lower activity in cultured keratinocytes, thereby suggesting the presence of upstream suppressor activity in the -616/-444 region. Since these results verified that the -216/-1 region contains the minimal promoter sequence for BH gene transcription, the nucleotide sequence thereof is shown in FIG. 18C. Since this sequence does not contain a TATA- or CCAAT-box, this gene was suggested to have housekeeping properties. On the other hand, several transcription factor binding sites, such as MZF-1, Sp-1, IRF-1/2 and GATA-1/2, were present in this core promoter region.

### Identification of Latent Cis Element Involved in BH Gene Regulation

In order to determine the latent cis element of the minimal promoter sequence involved in regulating transcription of BH gene expression, a new series of deletion mutant strains were constructed that were targeted at each cis element. Promoter activity was considerably down-regulated in the case of having deleted the MZF-1, Sp-1 and IRF-1/2 binding sites (FIG. 19A).

Moreover, an investigation was conducted as to whether or not these transcription factors are actually capable of bind to each presumed binding site. Thus, an electrophoretic mobility shift assay (EMSA) was carried out using nuclear extracts from cultured keratinocytes and biotinylated double-stranded oligonucleotide probes containing the MZF-1, Sp-1, GATA-1 or IRF-1/2 binding site. As shown in FIG. 19B, although Sp-1, MZF-1 and IRF-1/2 bound to target sites corresponding to BH promoter, GATA-1/2 did not bind. These results indicate that these binding sites of the -216 bp to -105 bp promoter region are essential for the cis element for BH transcription.

### Cytokine-Mediated Regulation of BH Gene Expression

Since BH is an NMF-producing enzyme, it has the possibility of being involved in the pathophysiology of AD. Accordingly, an investigation was conducted of the effects of Th1, Th2 and Th17 cytokines on BH gene expression. FIG. 20A indicates the dose-dependent down-regulation of BH mRNA expression by a Th1 cytokine, IFN-γ in proliferating keratinocytes. On the other hand, Th2 and Th17 cytokines did not demonstrate any significant effects on BH expression. Similar results were obtained with differentiated keratinocytes (data not shown). In order to clarify the role of IFN-γ in the regulation of BH gene expression, a promoter assay was carried out to identify cytokine response elements. As shown in FIG. 20B, IFN-γ down-regulated BH promoter activity in cultured keratinocytes transfected with pGL3-BH-616 containing IRF-1/2 binding sites between -131 and -120. IFN-γ no longer suppressed promoter activity after this sequence was deleted (FIG. 20B). In addition, in order to determine whether or not IRF-1/2 is an essential mediator of IFN-γ-induced down-regulation of BH, IRF-1 and IRF-2 gene expression was suppressed using small interfering RNA (siRNA). The activity of IFN-γ was significantly inhibited in cultured keratinocytes transfected with either IRF-1 or IRF-2 siRNA (40 nM) (FIG. 20C). These results strongly suggest that the IRF-1/2 binding sequence is essential for IFN-γ-induced down-regulation of BH gene expression.

### Expression of BH and Related Factors in Cultured Keratinocytes

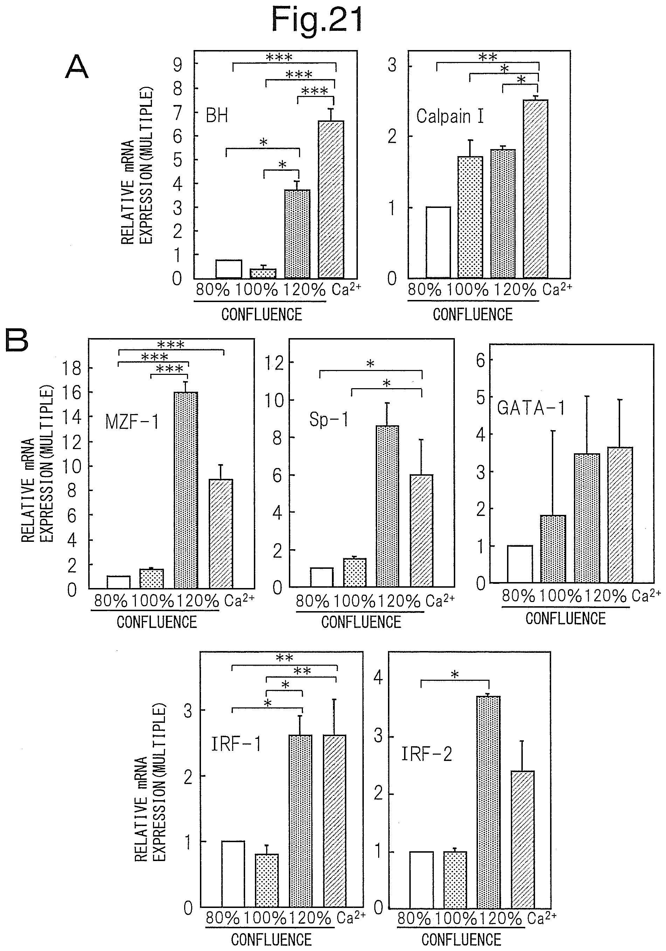

In order to investigate the mechanism of transcription regulation in the epidermis, the expression of BH, calpain-1 and presumed transcription factors was analyzed in proliferating and differentiated cells by real-time PCR. As shown in FIG. 21A, BH mRNA was up-regulated in differentiated keratinocytes, such as those obtained two days after confluence (by 3.6 times) or those cultured at a high calcium concentration (by 8.6 times) in comparison with proliferating keratinocytes. These results coincide with the promoter assay data (FIG. 18B). Similar results were obtained with respect to calpain-1 (up-regulated by about 2.5 times). In addition, the expression patterns of various transcription factors, such as MZF-1, Sp-1, GATA-1, IRF-1 and IRF-2, were investigated in cultured keratinocytes. As shown in FIG. 21B, these transcription factors were up-regulated in differentiated keratinocytes in parallel with BH expression. However, expression of GATA-1 mRNA was significantly lower in comparison with other factors (less than 1/32). GATA-1 is therefore not considered to play an important role in keratinocytes. Accordingly, it was suggested that BH is synthesized by a differentiation-dependent mode that is mediated by MZF-1 and Sp-1. The fact that IRF-1 and IRF-2 were also up-regulated as a result of being stimulated by differentiation indicates that BH expression is extremely sensitive to IFN-γ.

### Effects of Th1 and Th2 Cytokines on Expression of Presumed Transcription Factors

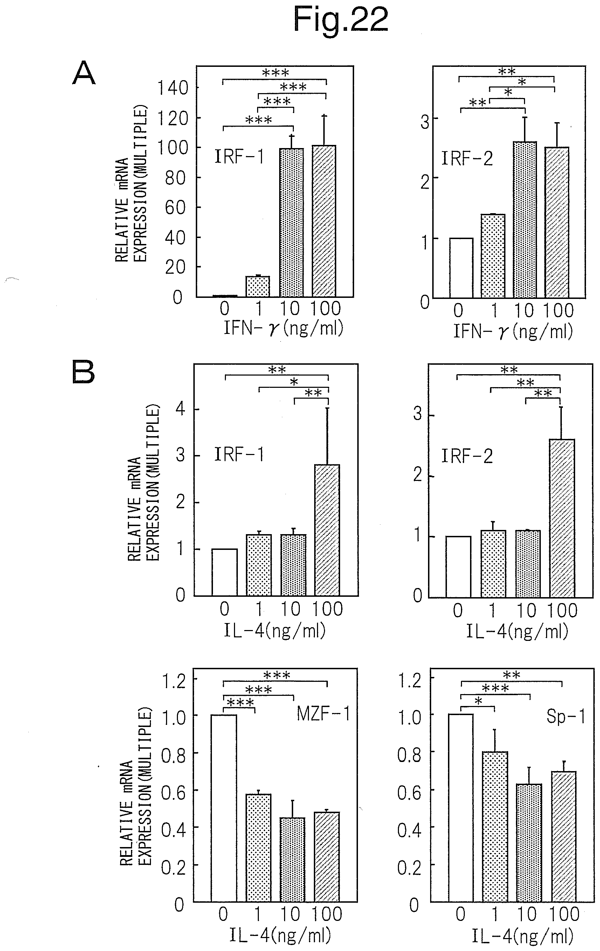

An investigation was conducted of the cytokine-dependent regulation of these transcription factors. FIG. 22A indicates that IFN-γ demonstrates potent dose-dependent up-regulation of IRF-1 mRNA expression. Similarly, IRF-2 expression was also up-regulated in the presence or IFN-γ. In contrast, expression of IRF-1 and IRF-2 was significantly amplified only in the presence of IL-4 at 100 ng/ml (FIG. 22B). It is interesting to note that both MZF-1 and Sp-1 were down-regulated most effectively in the presence of IL-4 at 10 ng/ml (FIG. 22C). These results suggest that BH expression is regulated by Th1 cytokine and Th2 cytokine directly and indirectly, respectively.

### Down-Regulation of BH in Atopic Dermatitis Skin

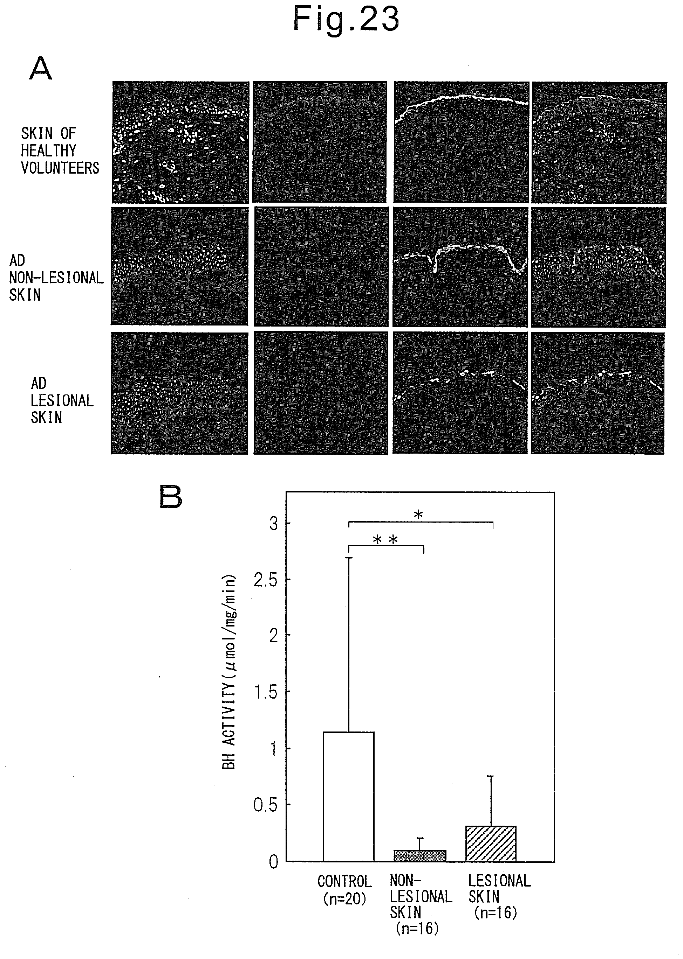

Although loss-of-function mutations of FLG are related to the pathogenic mechanism of AD, gene defects as well as disorders of the degradation pathway may also be related to the pathology of AD. Consequently, the following investigation was conducted for the localization of BH and filaggrin along with BH activity in lesional skin and non-lesional skin of AD patients. In the normal epidermis, double-staining with anti-BH antibody and anti-filaggrin antibody indicated simultaneous localization of BH and filaggrin in the upper epidermis, and particularly in the granular layer as previously reported (FIG. 23A). At higher magnifications, although BH was observed to be localized from the granular layer to the horny layer, filaggrin was clearly shown to be limited to granule cells. In contrast, BH expression dramatically decreased in lesional skin and non-lesional skin of AD patients (n=7) examined in this study. In all of these patients, even though significant staining was detected at all times, filaggrin was stained comparatively weakly (FIG. 23A). In addition to immunohistochemistry, BH activity was measured in keratinocyte extracts acquired from tape stripped samples obtained from 18 AD patients and 30 healthy volunteers. Extracts obtained from lesional skin and non-lesional skin of the AD patients demonstrated substantially lower BH activity in comparison with that of the healthy volunteers (decreasing by 27.1% and 8.8%, respectively) (FIG. 23B). These results demonstrated that BH is localized simultaneous to filaggrin, and that the activity thereof is lowered dramatically in the skin of patients suffering from AD.

### Discussion

In this study, the regulatory mechanism of BH gene expression was examined by cloning the promoter region and characterizing its function. In the promoter analysis, a region important for BH promoter activity was identified 16 bp upstream (FIG. 18B). In this region, the presumed MZF-1 and Sp-1 binding sites demonstrated significant effects on BH promoter activity (FIGS. 18C and 19A). It is interesting to note that Sp-1 and MZF-1 have also been reported to be involved as transcription factors in the regulation of PAD1, and important enzyme for initiation of filaggrin degradation. Sp-1 is a typical member of the Sp/Kruppel-like family of zinc finger proteins that function as transcription factors in mammalian cells. It is considered to be involved in nearly all aspects of cell function, including proliferation, apoptosis, differentiation and neoplastic transformation. In the human epidermis, Sp-1 is an important regulatory factor of genes participating in epidermal differentiation, including those of involucrin, loricrin, transglutaminase and PAD 1, 2 and 3. MZF-1 is a transcription factor belonging to the Kruppel family of zinc finger proteins, and is expressed in differentiated pluripotent hematopoietic cells and bone marrow progenitor cells. However, the function of MZF-1 in the regulation of transcription in mammalian epidermis has not been reported. MZF-1, Sp-1 and BH were found to be simultaneously up-regulated in differentiated keratinocytes in comparison with proliferating keratinocytes (FIG. 21B), thus demonstrating the role of BH in differentiation rather than a housekeeping role. Our results clearly indicated that these transcription factors function as activation factors for basic regulation of transcription of BH in keratinocytes undergoing terminal differentiation.

On the other hand, an investigation of cis-acting elements further defined the IRF-1/2 binding sites in this region. Direct binding of IRFs to the BH promoter region was confirmed using EMSA (FIG. 19B). Site-specific mutagenesis of this binding sequence brought about a significant decrease in BH promoter activity (FIG. 19A). Consequently, IRF-1/2 transcription factors are also most likely required for minimal promoter activity of BH gene under basic conditions. The IRF family consists of a group of transcription factors and at present, nine members of the IRF family (IRF-1 to IRF-9) have been identified in various cell types and tissues. These IRF molecules play a role in antiviral defense, immune response/regulation and cell growth regulation when stimulated by IFN-α, IFN-β and IFN-γ. IRF-1 and IRF-2 have been shown to function as agonists and antagonists involved in the regulation of numerous IFN-γ-induced genes. Interestingly, IFN-γ remarkably suppressed expression of BH mRNA (FIGS. 20A and 20B). In knockdown and site-specific mutagenesis analyses, the IRF-1/2 binding sites were confirmed to be involved in IFN-γ-mediated suppression of BH expression (FIGS. 20B and 20C). These results clearly indicate that IRF-1/2 are mediators of IFN-γ-mediated down-regulation of BH gene in human keratinocytes. On the other hand, Th2 cytokines, IL-4 and IL-13 did not demonstrate any direct action whatsoever during 24 hours of incubation (FIG. 20A). However, these Th2 cytokines significantly suppressed expression of activator molecules, MZF-1 and Sp-1. Accordingly, it is reasonable to think that Th2 cytokines negatively regulate BH expression.

In addition, BH was also shown to be dramatically down-regulated in lesional and non-lesional AD skin (FIGS. 23A and 23B). Although filaggrin mutations are major risk factors for barrier impairment-related diseases such as AD, in an analysis of such mutations, these mutations account for less than 50% of the occurrences of such diseases in Ireland and only account for no more than 20% of their occurrences in Japan. Thus, not only filaggrin synthesis disorders but also impairment of filaggrin degradation was hypothesized to be involved in disruption of the skin's barrier function. It is clear that decreased NMF brings about dry skin and causes progression of barrier disruption. Moreover, AD is widely known to be a Th2-polarized disease. However, recent reports have suggested that Th1 cytokines also play a role in AD. For example, "intrinsic AD" is characterized immunologically by low expression of IL-4, IL-5 and IL-13 and high expression of IFN-γ. In addition, a shift from Th1 to Th2 occurs during transformation from the acute phase to chronic phase in AD skin. Our results indicate the possibility of IFN-γ playing a more important role that was previously thought.

In conclusion, our results indicate that BH transcription in human epidermis is regulated by a dual mode. One of the pathways is under the control of keratinocyte terminal differentiation, while the other pathway is dependent on Th1 and Th2 cytokines. Since these pathways are interrelated, the balance between them is thought to easily shift towards down-regulation of BH expression. A decrease in BH brings about a shortage of NMF, and skin becomes dry or the skin's barrier function is disrupted as a result thereof. These results provide new insights into BH regulation and the mechanism of occurrence of AD.

### Experiment 7

### Screening for Pharmaceutical Agents and Herbal Medicines Demonstrating Bleomycin Hydrolase Production Promoting Effects

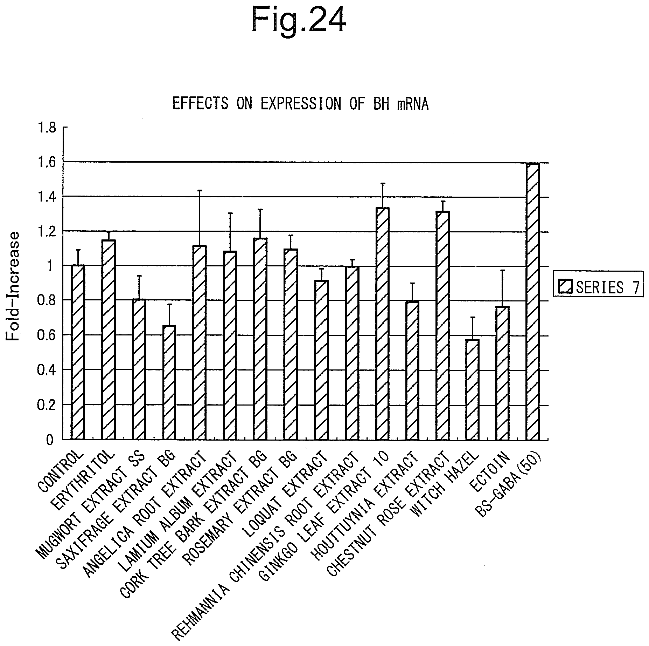

Human keratinocytes derived from normal foreskin (Cascade Biologics Inc., Portland, MD) were cultured for 24 hours at room temperature in the presence of each of the herbal medicine extracts or pharmaceutical agents (5 µg/ml to 50 µg/ml) shown in FIG. 24 in keratinocyte growth medium consisting of MCDB medium supplemented with epidermal growth factor (0.1 ng/ml), insulin (10 µg/ml), hydrocortisone (0.5 µg/ml), bovine pituitary extract (0.4%), gentamycin (50 µg/ml) and amphotericin B (50 ng/ml). 0.1% 1,3-butylene glycol was used as a control.

### RT-PCR

Total RNA (500 ng) isolated from the human keratinocytes cultured in the manner described above was reverse-transcribed using random hexamers and Superscript II RNase H-transcriptase (Gibco-BRL Corp., Gaithersburg, MD), followed by subjecting to PCR amplification using Taq DNA polymerase (Takara Corp., Kyoto, Japan) and the following primers. 40 amplification cycles were carried out consisting of 30 seconds at 94°C, 1 minute at 60°C and 1 minute at 72°C.

According to the results shown in FIG. 24, extracts such as chestnut rose extract (10 µg/ml), angelica root extract (10 ng/ml), cork tree bark extract (10 µg/ml), lamium album extract (10 µg/ml) and rosemary extract (10 µg/ml) as well as benzenesulfonyl GABA (50 µg/ml) and erythritol (50 µg/ml) can be understood to significantly increase expression of bleomycin hydrolase in comparison with the control. Thus, each of these extracts can be understood to promote expression of bleomycin hydrolase. The primers used in PCR are indicated below.
Forward primer: 5'-TGTGGTTTGGCTGTGATGTT-3' (SEQ ID NO: 13)
Reverse primer: 5'-GCACCATCCTGATCATCCTT-3' (SEQ ID NO: 14)
In addition, GAPDH was amplified by PCR for use as an internal control, and the primers used at that time are indicated below.
Forward primer: GGTGAAGGTCGGAGTCAACGGATTTGGTCG (SEQ ID NO: 15)
Reverse primer: TATTGGAACATGTAAACCATGTAGTTGAGG (SEQ ID NO: 16)

## Claims

1. A bleomycin hydrolase production promotor substance for use in a method of improving or preventing dry skin caused by atopic dermatitis due to the reduction of bleomycin hydrolase expression in skin, wherein the substance is chestnut rose extract.

## Patentansprüche

1. Substanz zur Förderung der Produktion von Bleomycin-Hydrolase für die Verwendung in einem Verfahren zur Verbesserung oder zur Vermeidung von trockener Haut, welche durch atopische Dermatitis hervorgerufen wird und durch eine Verringerung der Expression von Bleomycin-Hydrolase in der Haut bedingt ist, wobei es sich bei der Substanz um Kastanienrosenextrakt handelt.

## Revendications

1. Substance pouvant promouvoir la production de la bléomycine-hydrolase pour l'utilisation de la substance dans une méthode d'amélioration ou de prévention de peau sèche résultant d'une dermatite atopique engendrée par la diminution de l'expression de la bléomycine-hydrolase dans la peau, dans laquelle la substance est un extrait de rose châtaigne.
